# EUROPÄISCHE PATENTSCHRIFT

(11) **EP 2 026 872 B1**
(45) Veröffentlichungstag und Bekanntmachung des Hinweises auf die Patenterteilung: **16.09.2009**
(21) Anmeldenummer: 07725343.3
(22) Anmeldetag: 18.05.2007
(51) Int. Cl.: A61N 1/05

(54) **VORRICHTUNG ZUR TRANSKUTANEN AUFBRINGUNG EINES REIZES ODER ZUR TRANSKUTANEN ERFASSUNG EINES PARAMETERS**
DEVICE FOR TRANSCUTANEOUS APPLICATION OF A STIMULUS OR FOR TRANSCUTANEOUS RECORDING OF A PARAMETER
DISPOSITIF D'APPLICATION TRANSCUTANÉE D'UN STIMULUS OU DE DÉTECTION TRANSCUTANÉE D'UN PARAMÈTRE

(30) Priorität: 20.05.2006 DE 102006023824
(43) Veröffentlichungstag der Anmeldung: 25.02.2009
(73) Patentinhaber: cerboMed GmbH, 91052 Erlangen (DE)
(72) Erfinder: DIETRICH, Stefan, 91056 Erlangen (DE); FREITAG, Timo, 91052 Erlangen (DE)
(74) Vertreter: Gosdin, Michael
(86) Internationale Anmeldenummer: PCT/EP2007/004435
(87) Internationale Veröffentlichungsnummer: WO 2007/134804

(56) Entgegenhaltungen:
- US-A- 4 073 296
- US-A- 4 267 838
- US-A- 5 514 175
- US-A1- 2006 064 139

## Beschreibung

Die Erfindung betrifft eine Vorrichtung zur transkutanen Aufbringung eines Reizes oder zur transkutanen Erfassung eines Parameters auf die bzw. von der Hautoberfläche eines Wirbeltieres, insbesondere eines Menschen, wobei die Vorrichtung so ausgebildet ist, dass sie vollständig in einer Pinna (Ohrmuschel) des Wirbeltieres unterbringbar ist.

Vorrichtungen dieser Art sind insbesondere gebräuchlich, um therapeutisch den Nervus vagus (Vagus-Nerv) elektrisch zu stimulieren, um einen medizinischen Effekt zu erreichen. Hierzu ist es bei Vorrichtungen zur transkutanen Stimulation von Nerven oder Akupunkturpunkten bekannt, durch invasive oder nicht-invasive elektrische Reizung der Nerven bzw. des umgebenen Gewebes mittels Clipsystemen (ähnlich einer Wäscheklammer) oder stiftähnlichen Systemen oder unter Zuhilfenahme kleiner Nadeln, welche nach Verbindung mit einer Steuereinheit zusätzlich elektrisch gereizt werden, Einfluss auf die neuroelektrische oder sonstige Qualität und somit auf die Funktion der zu stimulierenden Nerven oder des Gewebe zu nehmen. Ziel einer solchen Vorgehensweise ist es meist, psychovegetative Veränderungen wie beispielsweise Stressabbau hervorzurufen.

Besondere wissenschaftliche Bedeutung haben einerseits die Stimulation von Nerven und Geweben im Bereich des Ohres, insbesondere die elektrische Stimulation sich dort befindlicher Anteile des Nervus vagus oder die Stimulation von Geweben und Nerven mittels kalorischer, magnetischer oder sonstiger Stimulationsqualitäten, sowie die Detektion verschiedener physiologischer, pathophysiologischer oder physikalischer Parameter wie beispielsweise der Körpertemperatur oder der Sauerstoffsättigung.

Insbesondere die nicht-invasive Stimulation des Vagus-Nervs, dem Hauptnerv des parasympathischen Nervensystems, welcher Hautafferenzen im Bereich des äußeren Ohres führt, wird derzeit für diagnostische und therapeutische Zwecke von neurodegenerativen Erkrankungen wie Alzheimer, Parkinson oder Epilepsie vereinzelt im wissenschaftlichen Schrifttum erwähnt, wobei auf Ventureyra ECG: "Transcutaneous vagus nerve stimulation for partial onset seizure therapy", Child's Nerv Syst (2002) 16:101-102, und auf Fallgatter AJ, Ehlis AC, Ringel T, Herrmann M: "Age effect on far field potentials from the brain stem after transcutaneous vagus nerve stimulation", Int J Psychophysiol (2005) 56:37-, 43, sowie Fallgatter AJ, Neuhauser B, Herrmann MJ, Ehlis AC, Wagener A, Scheuerpflug P, Reiners K, Riederer P: "Far field potentials from the brain stem after transcutaneous vagus nerve stimulation", J Neural Transm (2003) 110:1437-1443 hingewiesen wird. In der Arbeit von Ventureyra wird lediglich die Möglichkeit angedacht, elektrische Stimuli am Ohr zu applizieren, um damit epileptische Anfälle zu behandeln. Lösungsansätze für eine Applikationsvorrichtung werden nicht gegeben. In den Studien von Fallgatter et al. werden eine Stimulations- und eine Referenzelektrode auf ein Stück Kupferblech, welches mit Epoxidharz beschichtet ist, aufgebracht und mit einem Heftpflaster am Ohr befestigt, um so ebenfalls elektrische Stimuli zu diagnostischen Zwecken nach Ableitung eines elektroenzephaltigraphischen Signals (Himstrommessung) mit eine kommerziell erwerbbaren Gerät einzusetzen. Eine solche selbstgebaute Vorrichtung eignet sich nicht zur passgenauen Platzierung der Elektroden an der Ohrmuschel, da sie mit Klebeband befestigt werden muss und nicht der Form des äußeren Ohres nachempfunden ist.

Zur passgenauen Platzierung eines Sensors oder einer Sensoreinheit zum Erfassen von physiologischen, pathophysiologischen oder physikalischen Parametern bzw. zur elektrischen oder anders gearteten Stimulation von Nerven und Geweben an der Ohrmuschel oder im Gehörgang sind die folgenden beiden Vorrichtungen bekannt:

Die US 5 458 625 beschreibt eine Vorrichtung und ein Verfahren zur Stimulation dicht unter der Haut der Ohrmuschel befindlicher Nervenanteile des Nervus vagus zur Behandlung von Stress, Schmerzen oder zur Muskelrelaxation. Das Gerät beinhaltet ein Paar transkutaner (durch die Haut hindurch wirkender) Stimulationselektroden. Diese sind in Form von wäscheklammerförmigen Clips konstruiert und zur Anbringung an das Ohrläppchen geeignet. Über die Clipelektroden wird ein Stimulationsstrom beaufschlagt, welcher zur Nervenstimulation geeignet erscheint.

In **der** US 2003/195588 A1 wird ein multimodaler Nervenstimulator vorgeschlagen. Eine Vorrichtung, welche verschiedene Sensoren und ringförmige Elektroden beinhalten kann, muss tief zum Wirkungsort in den äußeren Gehörgang eingeführt werden. Beschrieben ist hier weiterhin, dass im äußeren Gehörgang Anteile des Nervus vestibularis stimuliert werden, welche positive Eigenschaften bei der Behandlung insbesondere von Epilepsie und Schwindel haben könnten. Die Vorrichtung sieht zudem auch eine der Anatomie des Gehörgangs angepasste Bauform vor.

Die US 4 267 838 offenbart eine Vorrichtung, die eine Otoplastik aufweist, die von einem externen Steuergerät mit Strom versorgt wird, um eine Akupressuranwendung vornehmen zu können. Zur Akupressur dient auch die Vorrichtung gemäß der US 4 073 296**.** Ähnliche Lösungen sind aus der US 2006/0064139 A1 und aus der US 5 514 175 bekannt.

Bei den genannten vorbekannten Lösungen haben sich folgende Umstände als nachteilig erwiesen:

Im wissenschaftlichen Schrifttum wird - wie oben aufgezeigt - die Stimulation des Tragus bzw. einem definierten anatomischen Anteil desselben erwähnt. Die beschriebenen wäscheklammerförmigen Clipsysteme ermöglichen keine Stimulation von zur Therapie insbesondere von Depressionen, Epilepsie, kardialer Symptomatik und weiteren neuropathologischen Krankheitszuständen relevanten Nervenanteilen im Bereich der Ohrmuschel. Hinzu kommt, dass die bei solchen Klippsystemen zur Überwindung des Hautwiderstandes benötigten verhältnismäßig hohen Spannungen von 80 Volt und mehr ein sehr hohes Verletzungsrisiko bei einer Applikation insbesondere am Tragus oder anderen Strukturen der Ohrmuschel darstellen.

Beschriebene Clipsysteme eignen sich aufgrund ihrer mechanischen Halteeigenschaften nicht zur Applikation während einer Körperbewegung. Es besteht die Gefahr, dass sich die Verbindungen lösen. Dies birgt wiederum ein Verletzungsrisiko für den Träger durch nun herabhängende Kabelverbindungen sowie die Gefahr eines Elektronikschadens in der Stimulationseinheit beispielsweise durch einen Kurzschluss in sich.

Die vorbekannte Vorrichtung eines multimodalen Neurostimulators beschreibt explizit ein Ohrstück, welches sich nur zum Einführen in den äußeren Gehörgang eignet. Bereits die Fertigung eines solchen Im-Kanal-Ohrstücks ist mit großen Schwierigkeiten verbunden. Hierzu ist aufgrund vorbekannter Verfahren insbesondere für eine individuelle Bauweise eine Ohrabformung mit einem Abformmaterial notwendig, um den anatomischen Verlauf des Gehörgangs möglichst detailgetreu nachbilden zu können. Eine solche Abformung ist mit verschiedenen Risiken verbunden, insbesondere einer Verletzung des Trommelfells sowie verschiedener Hautpartien des dort sehr empfindlichen Gewebes. Beim Vorliegen von otologischen Pathologien wie z.B. einer Ruptur des Trommelfells ist eine Abformung nochmals erschwert und kann nur vom Fachmann vorgenommen werden.

Das Gewebe des äußeren Gehörgangs ist sehr anfällig für Hautirritation, wobei es beim Einbringen von Fremdkörpern beispielsweise im Fall eines vorbekannten Neurostimulators vor allem als Langzeitwirkung zu Entzündungen und Allergien kommen kann.

Bei einer nicht-individuellen Ausführung besteht eine erhöhte Verletzungsgefahr der Gehörgangsstrukturen durch fehlende Passgenauigkeit.

Durch das sich im Gehörgang befindliche aufgezeigte Ohrstück wird gemäß dem genannten Dokument nur der Nervus vestibularis stimuliert. Dieser Nerv ist ein Teilast des Nervus vestibulocochlearis, welcher nur rein sensorische Faserqualitäten aufweist. Hierdurch wird eine Einflussnahme auf das Gleichgewichtsorgan eines Wirbeltiers und somit auf Schwindelerscheinungen, Übelkeit oder Raumorientierung möglich, nicht aber eine Reizung weiterer Nerven insbesondere des Nervus vagus.

Das Anbringen von Sensoren auf einem solchen Gehörgangspassstück, welches Hautkontakt mit dem äußeren Gehörgang aufweist, scheint sinnvoll. Es sind aber folgende Nachteile zu nennen: Das Hautgewebe des äußeren Gehörgangs produziert dauerhaft das Cerumen, umgangssprachlich auch Ohrenschmalz genannt. Dieses Sekret setzt sich auf der Oberfläche der Sensoren ab und verfälscht so die Messergebnisse. Zudem führt es zu einer starken und nachhaltigen Verunreinigung der Sensoren, was Auswirkungen auf die Lebensdauer eines solchen Systems haben dürfte. Zudem wird eine fehlende Verbindung zur Ohrmuschel, wo zahlreiche Parameter ebenfalls aber in vorteilhafter Weise abgegriffen werden können, sowie zur Umwelt als Träger zusätzlicher nützlicher Sensorinformationen als negativ angesehen.

Letztendlich wird die Gestaltung der Stimulationselektroden als nachteilig angesehen. Die beschriebene vorbekannte Lösung beschreibt ringförmig um das Ohrstück angelegte Elektrodenschleifen. Diese sind jedoch in ihrer Fläche begrenzt und erlauben keine Stimulation eines bestimmten Areals in nur einer Dimension sowie keine Stimulation von Anteilen der Ohrmuschel.

Für einen großflächigen therapeutischen und diagnostischen Einsatz der Methode der transkutanen Nervenstimulation am Ohr sowie der Messung verschiedener Parameter ist es wünschenswert, ein Therapie- sowie Sensorsystem in eine handliche, sichere möglichst individuell adaptierbare Vorrichtung zu integrieren, die jederzeit, unkompliziert, bequem und unauffällig getragen werden kann und sich insbesondere zum Befestigen an oder in der Ohrmuschel eignet.

Der Erfindung liegt daher die **Aufgabe** zugrunde, eine Vorrichtung der eingangs genannten Art so auszubilden, dass die eben aufgezeigten wünschenswerten Anforderungen befriedigt werden. Es soll eine Vorrichtung zur Applikation von Sensor- und Therapiesystemen am oder im Ohr eines Wirbeltiers geschaffen werden, die eine besonders effiziente und einfache Stimulation der sich in oder am Ohr befindlichen Nervenanteile insbesondere des Vagusnervs bzw. eine besonders effiziente und einfache Messung verschiedener Bio- und Umweltparameter ermöglicht und zwar in einfacher und quasi nicht sichtbarer Weise. Die Vorrichtung soll für den Patienten in besonders einfacher und vor allem sicherer Weise anwendbar sein und es insbesondere ermöglichen, gegebenenfalls schnell und sicher vom Körper entfernt werden zu können. Es soll dabei weiter eine effiziente Vorrichtung vorgeschlagen werden, die eine non-invasive Stimulation insbesondere der Vagusnervanteile ermöglicht und einen hohen Tragekomfort aufweist, welcher nicht zwingen über eine Fertigung bzw. Anpassung durch einen Fachmann erfordert. Weiterhin soll die Vorrichtung an Stellen eines Ohres mit potentiell geringem Störeinfluss durch Verschmutzung angebracht werden können. Weiterhin soll die Vorrichtung auf ein einfaches und sicheres Konstruktions- und Herstellungsverfahren, welches für eine großflächige Produktion geeignet ist, abstellen. Im Falle der Nervenstimulation wird angestrebt, diese effizient, ergonomisch günstig und für den Patienten leicht handhabbar zu machen.

Die **Lösung** dieser Aufgabe durch die Erfindung ist dadurch gekennzeichnet, dass die Vorrichtung ein vollständig in der Pinna unterbringbares, die Steuerungselektronik enthaltendes Gehäuse aufweist, wobei zur kraft- bzw. reibschlüssigen Fixierung der Vorrichtung in der Pinna am Gehäuse zwei gebogene, drahtförmige und federnd wirkende Abschnitte angeordnet sind.

Vorzugsweise hat die Vorrichtung zumindest abschnittsweise, insbesondere ausschließlich, eine C-förmige Gestalt. Die beiden gebogenen, drahtförmigen Abschnitte können an ihren Enden ein kugelförmiges Element aufweisen, wobei diese bevorzugt als Metallkugeln oder Flächenelektroden aus leitendem Kunststoff bzw. Karbonfasern ausgebildet sind, um als Elektroden zu fungieren.

Das Gehäuse kann zur Nervenstimulation eine Einrichtung zur Erzeugung eines elektrischen Reizes beinhalten und die beiden gebogenen, drahtförmigen Abschnitte können in ihrem Endbereich jeweils eine Elektrode zur Aufbringung des Reizes auf die Hautoberfläche aufweisen. Das Gehäuse und die beiden gebogenen, vorzugsweise drahtförmigen Abschnitte können so ausgebildet und anordenbar sein, dass die Elektroden im Bereich des bzw. nahe des Tragus zu liegen kommen. Die beiden gebogenen, vorzugsweise drahtförmigen Abschnitte sind mit Vorteil so ausgebildet und anordenbar, dass sie auf der Hautoberfläche in regio oder ober- bzw. unterhalb der Hautafferenzen des Nervus vagus (Vagusnerv) zu liegen kommen.

Das Gehäuse kann auch alternativ oder additiv eine Einrichtung zur Erfassung eines Parameters, nämlich eines physiologischen, eines pathophysiologischen oder eines physikalischen Parameters, beinhalten und die beiden gebogenen, drahtförmigen Abschnitte können als federelastische Halterungen ausgebildet sein.

Das Gehäuse und/oder die gebogenen, vorzugsweise drahtförmigen Abschnitte können aus verformbarem Material bestehen. Dabei ist gemäß einer Weiterbildung daran gedacht, dass das Gehäuse aus einer Kombination zweier Materialien besteht, insbesondere aus einem weichen und aus einem harten Material.

In der Vorrichtung kann eine wiederaufladbare Batterie angeordnet sein. Schließlich kann die Vorrichtung einen Kopfhörer aufweisen.

Die vorgeschlagene Vorrichtung weist also vorzugsweise mindestens einen der Form und Größe des äußeren Ohres angepassten Festkörper auf, in dem mindestens ein Sensor oder eine Sensoreinheit zum Erfassen von physiologischen, pathophysiologischen oder physikalischen Parametern oder mindestens eine Stimulationselektrode und mindestens eine Referenzelektrode oder mindestens ein Applikator sonstiger Stimulationsqualitäten, so zu liegen kommen, dass der mindestens eine Sensor oder die mindestens eine Stimulationselektrode und mindestens eine Referenzelektrode oder der mindestens eine Applikator sonstiger Stimulationsqualitäten Kontakt mit der Hautoberfläche des Ohres eines Wirbeltiers zur Messung von Parametern oder zur Beaufschlagung eines elektrischen Stroms oder sonstiger Stimulationsqualitäten nach Verbindung mit einer in den Festkörper integrierten oder externen Steuereinheit aufweist oder in sonstiger mittelbarer oder unmittelbarer Verbindung mit der Umgebung steht. Dabei ist die Vorrichtung insbesondere zur Stimulation des Nervus vagus im Bereich der Ohrmuschel ausgebildet oder geeignet.

Der Festkörper der Vorrichtung besteht bevorzugt aus einem Kunststoff welcher entweder starre oder flexible und verformbare Eigenschaften aufweist. Er kann an verschiedenen Stellen der Ohrmuschel, d. h. an der Vorderseite an unter sich gehenden Stellen, Vertiefungen und Erhöhungen befestigt sein.

Der Festkörper kann vorgeformt sowie individuell gestaltet sein. Im ersten Fall ist es für den Träger der Vorrichtung möglich, den Festkörper durch Verformen z. B. unter Wärmezufuhr an die individuellen Begebenheiten seiner Ohrmuschel anzupassen. Im zweiten Fall wird der Festkörper nach einer Ohrabformung durch einen Fachmann mit einem Abformmaterial vorzugsweise Silikon oder Alginat z. B. im Negativ-Positiv-Verfahren individuell hergestellt. Es kann zudem vorgesehen sein, den Festkörper im CAD/CAM-Verfahren herzustellen.

Für optimalen Tragekomfort sowie zur Verbesserung des Hautkontaktes der mindestens einen Stimulationselektrode und der mindestens einen Referenzelektrode sowie des mindestens einen Sensors kann der Festkörper aus einer Materialkombination bestehen. Dies kann eine Kombination aus hart bleibendem und weich bleibendem Kunststoff sein.

Eine Weiterbildung sieht hierzu sieht vor, in der Region an der Innenseite des Tragus hart bleibenden Kunststoff zu verwenden, in den die Elektroden und/oder Sensoren eingebettet sind. Von den Enden des hart bleibenden Abschnitts können krallenförmige Fortsätze wegziehen, welche aus weich bleibendem verformbaren Material bestehen und in der Concha sowie in der Hemiconcha superior Halt finden. Der Festkörper dieser Weiterbildung kann zudem entweder vollständig aus hart bleibendem oder weich bleibendem Material bestehen.

Weiterhin kann vorgesehen sein, in der Region an der Innenseite des Tragus hart bleibendes Material zu verwenden, in das die Elektroden und/oder Sensoren eingebettet sind und die krallenförmigen Haltefortsätze durch verformbare drahtähnliche Haltevorrichtungen zu ersetzen. Diese können mit einem anderen Material überzogen sein und an ihren Enden einen kugel- oder flächenähnlichen Fortsatz aufweisen.

Eine andere Weiterbildung sieht vor, dass der Festkörper den gesamten Bereich der Concha und der Hemiconcha superior ausfüllt.

In den Festkörper sind gemäß einer Weiterbildung mindestens eine Stimulationselektrode und/oder mindestens eine Referenzelektrode zur Elektrostimulation eingebracht. Die Elektroden können die Oberfläche des Festkörpers durchbrechen. Sie dienen als Kontaktstellen mit der Körperoberfläche. Die Kontaktstellen können aus Metallkugeln gebildet werden. Sie können auch durch flache Oberflächenelektroden gebildet werden. Weiterhin ist es möglich, dass die Kontaktstellen durch ein Element aus einem Material mit elektrischer Oberflächenleitfähigkeit gebildet werden. Über eine externe Steuereinheit, die nicht zentraler Gegenstand dieser Erfindung ist, kann auf die Elektroden ein Strom beaufschlagt werden.

Eine Weiterbildung sieht vor, die Elektroden durch Signalgeber für eine magnetische, kalorische, taktile oder sonstige Stimulationsqualität zu ersetzen. Signalgeber sonstiger Stimulationsqualitäten können auch mit den Elektroden kombiniert werden oder gemeinsam vorkommen.

Weiterhin ist vorgesehen, dass die Vorrichtung mindestens einen Sensor zur Messung physiologischer, pathophysiologischer oder physikalischer oder chemischer Parameter des Trägers der Vorrichtung oder der Umwelt aufweist. Hierbei kann es sich beispielsweise um einen Temperatursensor, einen Bewegungssensor, einen Pulssensor, einen Blutdrucksensor, einen Blutflusssensor, einen Pulsoximetriesensor oder einen pH-Sensor handeln. Zudem sind Sensoren möglich, welche verschiedene Umweltparameter messen können und die Ergebnisse an eine Steuereinheit weitergeben.

Wird das Ohrstück eingesetzt, berühren die Elektroden und Sensoren die Hautoberfläche der Ohrmuschel bzw. sind Sensoren sicher im Bereich der Ohrmuschel fixiert und können so dort befindliche Muskel- und Nervenpartien insbesondere der Vagusnerv stimuliert werden, sowie verschiedene Bio- und Umweltparameter gemessen werden.

In den Festkörper kann weiterbildungsgemäß eine Batterie oder ein Akkumulator untergebracht sein, welche die Stromversorgung für sog. aktive Sensoren sicherstellen sollen.

Es kann vorgesehen sein, dass die Vorrichtung Anschlussstellen für einen akustischen Signalgeber aufweistder (Hierbei kann es sich beispielsweise um einen Tinnitusnoiser bzw. Tinnitusmasker handeln), im Sinne der oben genannten Ausführungen in die Vorrichtung integriert ist.

Mit dem Erfindungsvorschlag wird also eine Vorrichtung insbesondere zur Applikation von verschiedenen Stimulations- sowie Sensorqualitäten in der Ohrmuschel möglich, wobei eine in der Ohrmuschel insbesondere an der Innenseite des Tragus platzierte Stimulationselektrode zur transkutanen Stimulation des Ramus auricularis des Vagusnervs dient. Hiermit kann über eine Stimulationseinheit, die nicht zentraler Gegenstand dieser Erfindung ist, Einfluss auf dessen Funktion genommen werden. Die vorbekannten nicht-invasiven Nervenstimulationsverfahren und -vorrichtungen mittels Stromanwendung dienen der peripheren Nerven- und Muskelreizung insbesondere zur Schmerztherapie (TENS - transkutane elektrischen Nervenstimulation), zum Muskeltraining (EMS - elektrische Muskelstimulation) oder zur Elektroakupunktur. Keine der hier vorbekannten Vorrichtungen ist dazu vorgesehen und geeignet, die Ströme über eine erfindungsgemäße Applikationshilfe zu platzieren.

Die Applikationsvorrichtung ist vor allem bei der Therapie von Depressionen und Ermüdungszuständen sowie Epilepsie, kardialen Symptomen sowie weiteren von großem Vorteil.

Vorrichtungen zur Applikation von Stimulationsqualitäten in Ohrmuschel existieren bisher wie aufgezeigt nur vereinzelt in unvorteilhafter Weise. Hier schafft die Erfindung Abhilfe.

In der Zeichnung sind Ausführungsbeispiele der Erfindung dargestellt. Es zeigen:
- Fig. 1: die anatomischen Verhältnisse des äußeren Ohres in der Schnittdarstellung,
- Fig. 2: verschiedene anatomische Bereiche der Pinna (Ohrmuschel),
- Fig. 3: die Ohrmuschel mit Angabe der für die Befestigung der erfindungsgemäßen Vorrichtung in Frage kommenden Bereiche,
- Fig. 4: schematisch das Schaltbild einer Vorrichtung zur Applikation verschiedener Stimuli an der Ohrmuschel sowie zur Detektion verschiedenster Parameter mittels Sensoren,

- Fig. 5a/b: jeweils ein Ohrstück mit entsprechenden Elektroden und Sensoren schematisch sowie in-situ, .
- Fig. 6: ein Ausführungsbeispiel einer Applikationsvorrichtung, welche die gesamte Concha bedeckt,
- Fig. 7: ein Ausführungsbeispiel einer krallenförmigen Vorrichtung,
- Fig. 8: ein Ausführungsbeispiel eines Festkörpers mittels Drahtbefestigung und
- Fig. 9: ein Ausführungsbeispiel der erfindungsgemäßen Vorrichtung mit einer C-förmigen bzw. bohnenförmigen Ausgestaltung zur Vagüsnerv-Stimulation.

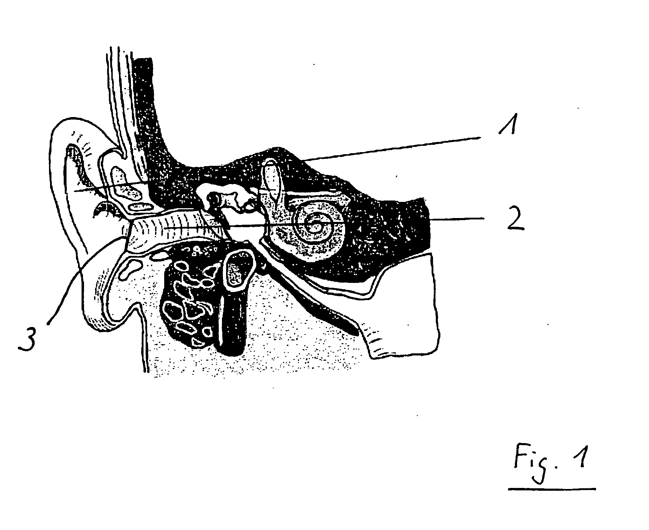

Die Fig. 1 gibt einen anatomischen Überblick zur Differenzierung zwischen der Pinna (Ohrmuschel) und dem äußeren Gehörgang. Das äußere Ohr setzt sich aus zwei Kompartimenten zusammen: der Ohrmuschel 1 und dem äußeren Gehörgang 2. Die anatomische Grenze zwischen beiden Einheiten ist der Porus acusticus externus 3, der Eingang zum äußeren Gehörgang 2.

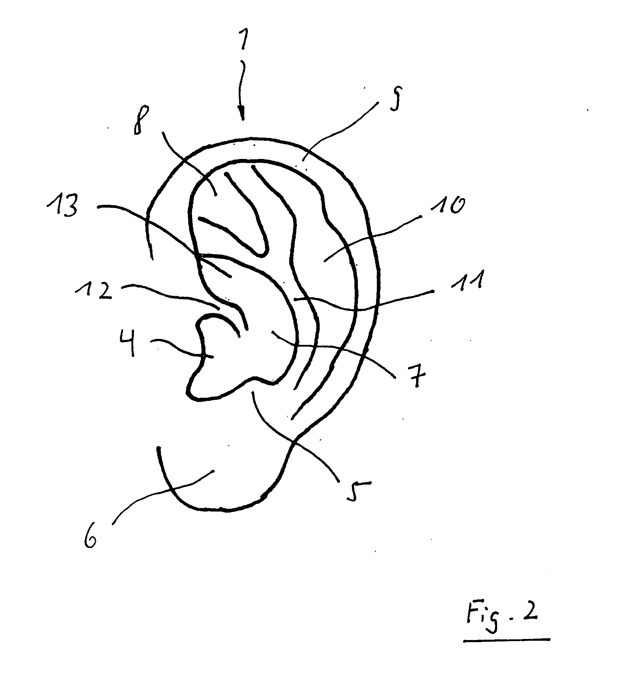

Die Ohrmuschel weist zahlreiche anatomisch benannte Anteile auf. Zur Visualisierung wird hier auf Fig. 2 hingewiesen. In dieser Figur sind wichtige anatomische Strukturen der Ohrmuschel gekennzeichnet. Diese Strukturen werden wichtig, wenn man sich die Innervation der Ohrmuschel betrachtet. So ist der Tragus 4 bzw. seine Innenseite der einzige Ort an der Ohrmuschel, an dem Anteile des Nervus vagus als Rami auriculares N. vagi dicht unter der Hautoberfläche verlaufen. Diese Nervenanteile eignen sich für eine elektrische Nervenstimulation durch eine erfindungsgemäße Vorrichtung zur therapeutischen und diagnostischen Zwecken.

In Fig. 2 angedeutet bzw. beziffert sind der Tragus 4, der Antitragus 5, das Ohrläppchen 6, die Concha 7, die Fossa triangularis 8, die Helix 9, die Scapha 10, die Anthelix 11, das Crus helicis 12 und die Hemiconcha superior 13.

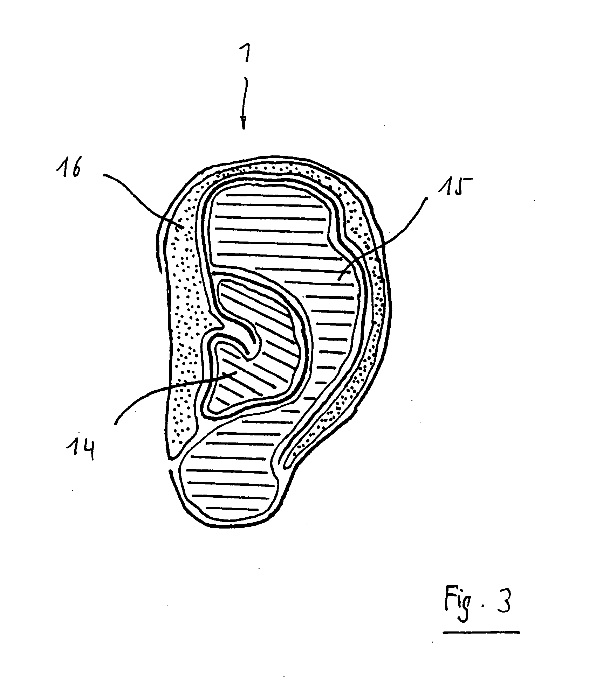

Aus Fig. 3 geht hervor, dass eine Vielzahl der Kontaktbereiche der Ohrmuschel mit der Applikationsvorrichtung für Elektroden und Sensoren in Frage kommt. Es wird auf die schraffiert und gepunktet markierten Kontaktbereiche 14 bis 16 hingewiesen. Nicht gezeigt sind Kontaktbereiche auf der Hinterseite der Ohrmuschel, die aber ebenfalls in Frage kommen.

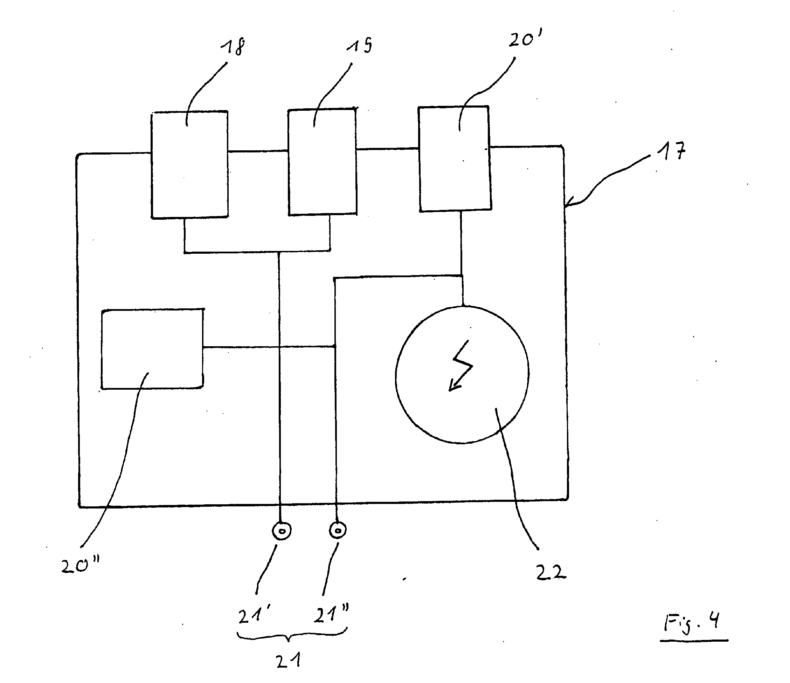

In Fig. 4 ist das Schaltbild einer Vorrichtung 17 zur Applikation verschiedener Stimulations- und Sensorqualitäten insbesondere zur transkutanen Vagusnervstimulation skizziert. Die Vorrichtung 17 besteht grundsätzlich aus mindestens einer Stimulationselektrode 18 und mindestens einer Referenzelektrode 19, wobei die Referenzelektrode 19 auch außerhalb der Vorrichtung 17 angebracht sein kann. Die Stimulation eines Nerven oder Gewebes erfolgt über die Stimulationselektrode 18. Als elektrischer Referenzpunkt dient die Referenzelektrode 19. Elektroden zur transkutanen Stimulation sind bekannt, kommerziell erhältlich bzw. leicht herzustellen. Die Stimulationsparameter können von einem externen Gerät, welches mit der Vorrichtung 17 über eine Anschlusseinheit 21, die in einen Anschluss für Stimulationselektroden 21' und einen Anschluss für Sensoren 21" aufgespalten sein kann, verbunden wird, beaufschlagt werden. Anschlüsse dieser Art sind bekannt und universell einsetzbar, so dass die Vorrichtung 17 mit verschiedenen externen Stimulationseinrichtungen verbunden werden kann. Auch das Anbringen spezifischer Anschlüsse 21' und 21", was dazu führt, dass die Vorrichtung 17 nur mit einem speziellen externen Gerät verbunden werden kann, ist möglich.

Weiter besteht die Vorrichtung 17 aus mindestens einem Sensor 20' oder 20". Der Sensor 20' weist direkten Kontakt mit der anliegenden Hautoberfläche auf. So können verschiedene Gewebeparameter wie beispielsweise Blutflussgeschwindigkeit oder die Blutsauerstoffsättigung bestimmt werden. Der Sensor 20' kann aber auch Kontakt mit der Umwelt aufweisen und dort verschiedene Parameter wie Temperatur oder Luftfeuchtigkeit bestimmen. Denkbar sind verschiedenste Sensorqualitäten. Ein anderer Sensor 20" weist keinen Kontakt mit der Außenwelt auf sondern ist in die Vorrichtung 17 fest integriert. Sensoren verschiedenster Art sind vorbekannt und kommerziell erhältlich.

Die Vorrichtung 17 kann zudem eine Stromquelle 22 in Form einer Batterie oder eines Akkumulators, welcher in vorteilhafter Ausführung induktiv oder mit Solarenergie aufgeladen werden kann, enthalten. Die Stromquelle 22 dient Sensoren 20' und 20", welche eine direkte aktive Stromversorgung benötigen, welche nicht durch eine von extern angeschlossene Stimulationseinheit gewährleistet ist.

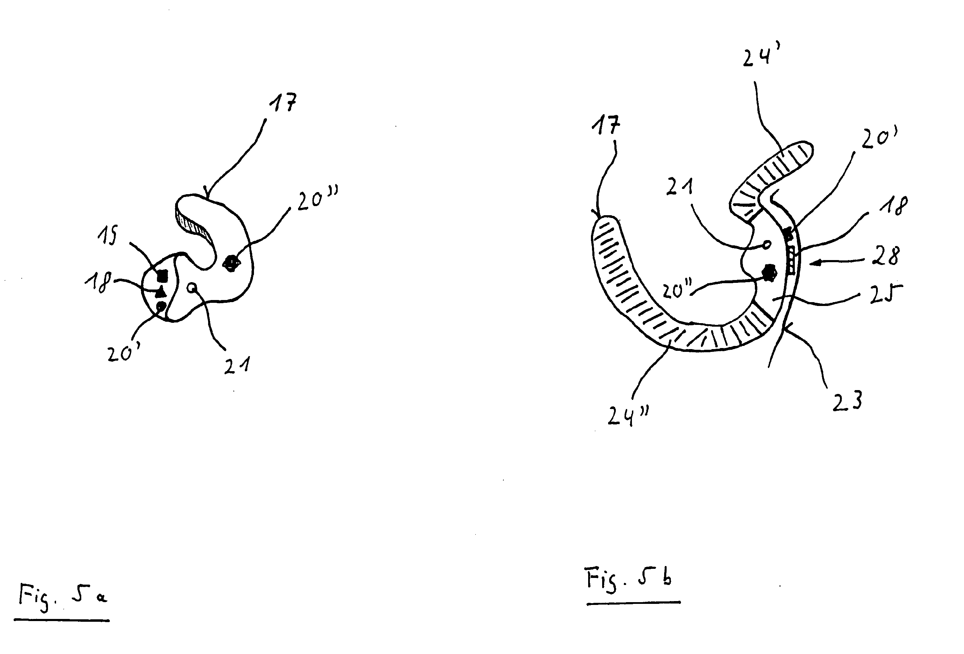

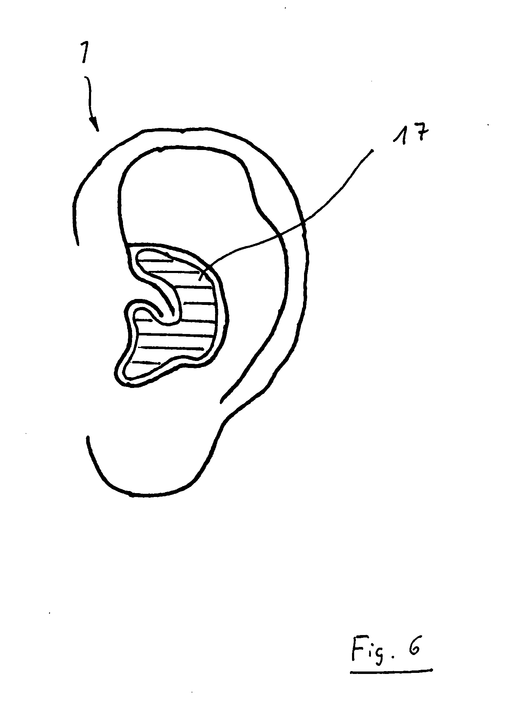

Die Fig. 5a zeigt ein Ausführungsbeispiel der Vorrichtung 13 welches nicht beansprucht wird, mit Anordnungsausführung der Elektroden 18 und 19, Sensoren 20' und 20" sowie dem Anschluss 21' bzw. 21". Der Festkörper der Vorrichtung 17 bedeckt hier, wie ebenfalls in Fig. 6 vereinfacht schraffiert dargestellt, den gesamten Bereich der Concha.

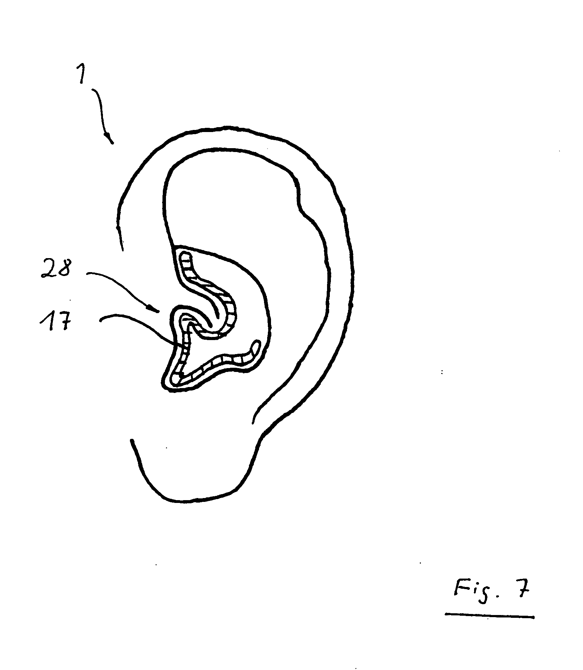

Ein Ausführungsbeispiel Fig. 5b, welches ebenfalls nicht beansprucht wird, sieht vor, den Festkörper der Vorrichtung 17 aus zwei Materialien herzustellen, welche unterschiedliche Festigkeitswerte und Eigenschaften aufweisen. So kann der unschraffierte Bereich 25 aus einem relativ harten, nicht verformbaren Material bestehen, in das mindestens die Stimulationselektrode 18, sowie die Sensoren 20' und 20" und eine Anschlusseinheit 21 eingelassen sind. Die schraffierten Bereiche 24' und 24" werden vorzugsweise aus einem weicheren, unter bestimmten Bedingung wie z.B. unter Wärmezufuhr verformbaren Material gebildet. So wird eine Anpassung an die individuelle Form der Ohrmuschel, im gezeigten Ausführungsbeispiel Fig. 5b sowie Fig. 7 an die Krümmung der Concha 7 und der Hemiconcha superior 13, möglich. Auf diese Weise ist eine individuelle Anpassung durch einen Fachmann nicht mehr möglich. Durch das Verformen entsteht im Festkörper der Vorrichtung 17 eine mechanische Spannung, sodass die mindestens eine Stimulationselektrode 18 sowie der mindestens eine Sensor 20' Kontakt mit der Hautoberfläche 23 haben. Hierdurch wird eine Stimulation durch einen auf die Stimulationselektrode 18 beaufschlagten Strom bzw. eine Messung eines Parameters über einen Sensor 20' möglich und gewährleistet. Zudem entsteht durch eine solche Bauweise ein hoher Tragekomfort unter Vermeidung weiter Wege zu einem Fachmann.

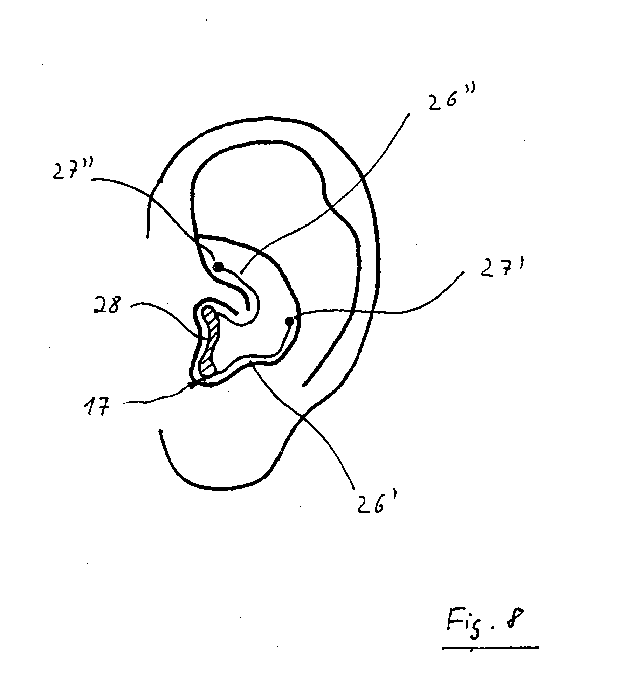

Das in Fig. 8 gezeigte, erfindungsgemäße Ausführungsbeispiel sieht vor, die in Fig. 5b gezeigten schraffierten, aus temporär weich bleibendem Material hergestellten Fortsätze 24' und 24" durch verformbare Drahtelemente 26' und 26" zu ersetzen. Hierdurch ergeben sich produktionsbedingte Vorteile, da das Verbinden zweier Kunststoffe mit unterschiedlichen Eigenschaften mit erhöhtem Produktionsaufwand verbunden ist. Dahingegen erscheint das mechanische Einlassen von verformbaren Drahtelementen 26' und 26" vorteilhaft. Die Drahtelemente können zum Schutz mit einer Ummantelung versehen sein. Zudem können sie zur Vorbeugung von Verletzungen sowie zur besseren Handhabung an ihren Enden kugelförmige Elemente 27' und 27" aufweisen. Im Ausführungsbeispiel gemäß Fig. 8 ist die Stimulationseinheit im Gehäuse 28 untergebracht, wobei die Drahtelemente 26' und 26" lediglich zur Halterung dienen. Erfindungsgemäß umfasst das Gehäuse 28 auch die Steuerungselektronik.

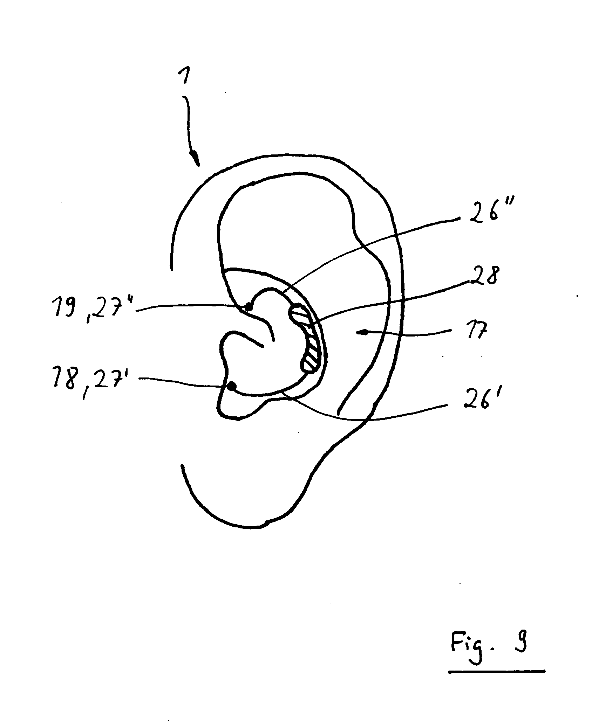

Aus Fig. 9 ist ein besonders bevorzugtes Ausführungsbeispiel ersichtlich, das die Vorrichtung 17 für die Vagusnerv-Stimulation zeigt. In der Ohrmuschel 1 vollständig untergebracht ist die Vorrichtung zur transkutanen Aufbringung eines elektrischen Reizes. Sie hat eine C-förmige Gestalt, wobei ein Gehäuse 28 - also ein Festkörper gemäß obiger Nomenklatur - zwei gebogene, drahtförmige Abschnitte 26', 26" aufweist, die sich vom Gehäuse 28 weg erstrecken. An den Enden weisen die beiden drahtförmigen Abschnitte 26', 26" Elektroden 18, 19 in Form metallischer, kugelförmiger Elemente 27', 27" auf.

Im Ausführungsbeispiel gemäß Fig. 9 ist die Stimulationseinheit samt Steuerungselektronik und Stromversorgung in dem bohnen- oder nierenförmigen Gehäuse 28 untergebracht, wobei hier die Drahtelemente 26' und 26" nicht nur zur Halterung dienen. Vielmehr verlaufen in diesen die Kabel zu den als Elektroden 18, 19 wirkenden Elementen 27', 27'. Die gesamte Vorrichtung 17 weist hier eine C-förmige Gestalt auf.

Die Vorrichtung 17 kann damit leicht und vollständig in der Ohrmuschel 1 befestigt werden, wobei die drahtförmigen Abschnitte 26', 26" federnd wirken und dadurch die gesamte Vorrichtung 17 gut in der Ohrmuschel 1 kraft- bzw. reibschlüssig fixieren.

Die beschriebene Vorrichtung wirkt mit einer Steuervorrichtung zusammenwirken, welche in des Pinna untesgekracht ist. Nicht beansprucht sind Ausgestaltungen, wonach diese Steuervorrichtung als hinter dem Ohr getragenes Gerät ausgeführt ist, oder, dass die Steuervorrichtung ein von Hand getragenes Gerät ist, wobei eine drahtlose Verbindung zwischen der Vorrichtung in der Ohrmuschel und der Steuervorrichtung herstellbar ist.

Die bevorzugte Ausführung der beinnaltet also eine Vorrichtung zur Applikation von Sensor- und Therapiesystemen im Ohr eines Wirbeltiers, die mindestens einen der Form und Größe des äußeren Ohres angepassten Festkörper aufweist, in dem mindestens ein Sensor oder eine Sensoreinheit zum Erfassen von physiologischen, pathophysiologischen oder physikalischen Parametern und mindestens eine Stimulationselektrode und mindestens eine Referenzelektrode oder mindestens ein Applikator sonstiger Stimulationsqualitäten, welche in der Lage sind einen Stimulus an die Hautoberfläche abzugeben, so zu liegen kommen, dass der mindestens eine Sensor oder die mindestens eine Stimulationselektrode und mindestens eine Referenzelektrode oder der mindestens eine Applikator sonstiger Stimulationsqualitäten Kontakt mit der Hautoberfläche des Ohres des Wirbeltiers zur Messung von Parametern oder zur Beaufschlagung eines elektrischen Stroms oder sonstiger Stimulationsqualitäten nach Verbindung mit einer internen Steuereinheit aufweist

Mit dem Erfindungsvorschlag wird eine Möglichkeit zur Applikation von Sensor- oder Therapiesystemen geschaffen, welche ihre Information aus bzw. von der Hautoberfläche oder am räumlichen Ort des Ohres eines Wirbeltiers beziehen oder dort Stimuli verschiedener Qualität platzieren können und zudem in oder an einen individuellen oder nicht-individuellen Ohreinsatz, welcher in der Ohrmuschel zum liegen kommt, eingearbeitet oder in sonstiger Weise daran befestigt sind.

### Bezugszeichenliste:

- 1: Ohrmuschel (Pinna)
- 2: äußerer Gehörgang
- 3: Porus acusticus externus
- 4: Tragus
- 5: Antitragus
- 6: Ohrläppchen
- 7: Concha
- 8: Fossa triangularis
- 9: Helix
- 10: Scapha
- 11: Anthelix
- 12: Crus helicis
- 13: Hemiconcha superior
- 14: Kontaktbereich
- 15: Kontaktbereich
- 16: Kontaktbereich
- 17: Vorrichtung zur Applikation von Elektroden und Sensoren in der Ohrmuschel
- 18: Stimulationselektrode
- 19: Referenzelektrode
- 20': Sensor
- 20": Sensor
- 21: Anschlusseinheit
- 21': Anschluss
- 21 ": Anschluss

- 22: Batterie oder Akkumulator
- 23: Hautoberfläche
- 24': Haltefortsatz / gebogener, drahtförmiger Abschnitt
- 24": Haltefortsatz / gebogener, drahtförmiger Abschnitt
- 25: Einheit aus hart bleibendem Material
- 26': Drahthaltefortsatz
- 26": Drahthaltefortsatz
- 27': kugelförmiges Element
- 27": kugelförmiges Element
- 28: Gehäuse (Festkörper)

## Patentansprüche

1. Vorrichtung zur transkutanen Aufbringung eines Reizes oder zur transkutanen Erfassung eines Parameters auf die bzw. von der Hautoberfläche eines Wirbeltieres, insbesondere eines Menschen, wobei die Vorrichtung (17) so ausgebildet ist, dass sie vollständig in einer Pinna (Ohrmuschel, 1) des Wirbeltieres unterbringbar ist,
**dadurch gekennzeichnet,**
**dass** die Vorrichtung (17) ein vollständig in der Pinna (1) unterbringbares, die Steuerungselektronik enthaltendes Gehäuse (28) aufweist, wobei zur kraft- bzw. reibschlüssigen Fixierung der Vorrichtung (17) in der Pinna (1) am Gehäuse (28) zwei gebogene, drahtförmige und federnd wirkende Abschnitte (24', 24", 26', 26") angeordnet sind.

2. Vorrichtung nach Anspruch 1, **dadurch gekennzeichnet, dass** sie zumindest abschnittsweise eine C-förmige Gestalt aufweist.

3. Vorrichtung nach Anspruch 2, **dadurch gekennzeichnet, dass** sie ausschließlich eine C-förmige Gestalt aufweist.

4. Vorrichtung nach einem der Ansprüche 1 bis 3, **dadurch gekennzeichnet**, die beiden gebogenen, drahtförmigen Abschnitte (24', 24", 26', 26") an ihren Enden ein kugelförmiges Element (27', 27") aufweisen.

5. Vorrichtung nach Anspruch 4, **dadurch gekennzeichnet, dass** die kugelförmigen Elemente (27', 27") als Metallkugeln ausgebildet sind.

6. Vorrichtung nach einem der Ansprüche 1 bis 5, **dadurch gekennzeichnet, dass** das Gehäuse (28) eine Einrichtung zur Erzeugung eines elektrischen Reizes beinhaltet und die beiden gebogenen, drahtförmigen Abschnitte (24', 24", 26', 26") in ihrem Endbereich jeweils eine Elektrode (18, 19) zur Aufbringung des Reizes auf die Hautoberfläche aufweisen.

7. Vorrichtung nach Anspruch 6, **dadurch gekennzeichnet, dass** das Gehäuse (28) und die beiden gebogenen, drahtförmigen Abschnitte (24', 24", 26', 26") so ausgebildet und anordenbar sind, dass die Elektroden (18, 19) im Bereich des bzw. nahe des Tragus (4) zu liegen kommen.

8. Vorrichtung nach Anspruch 7, **dadurch gekennzeichnet, dass** die beiden gebogenen, drahtförmigen Abschnitte (24', 24", 26', 26") so ausgebildet und anordenbar sind, dass sie auf der Hautoberfläche oberhalb des Nervus vagus (Vagusnerv) zu liegen kommen.

9. Vorrichtung nach einem der Ansprüche 1 bis 5, **dadurch gekennzeichnet, dass** das Gehäuse (28) eine Einrichtung zur Erfassung eines Parameters, nämlich eines physiologischen, eines pathophysiologischen oder eines physikalischen Parameters, beinhaltet und die beiden gebogenen, drahtförmigen Abschnitte (24', 24", 26', 26") als federelastische Halterungen ausgebildet sind.

10. Vorrichtung nach einem der Ansprüche 1 bis 9, **dadurch gekennzeichnet, dass** die gebogenen, drahtförmigen Abschnitte (24', 24", 26', 26") aus verformbarem Material bestehen.

11. Vorrichtung nach einem der Ansprüche 1 bis 10, **dadurch gekennzeichnet, dass** in der Vorrichtung eine wiederaufladbare Batterie (22) angeordnet ist.

## Claims

1. Apparatus for transcutaneous application of a stimulus or for transcutaneous measurement of a parameter on or from the skin surface of a vertebrate, in particular a human, wherein the apparatus (17) is designed so that it can be accommodated completely in a pinna (external ear, 1) of the vertebrate,
**characterized in that**
the apparatus (17) has a housing (28) which is arrangable completely in the pinna (1) and in which the electronic control (28) is accommodated, wherein two curved, wire-shaped sections (24', 24", 26', 26") having a spring action are arranged at the housing (28) for a nonpositively or frictionally fixing of the apparatus (17) in the pinna (1).

2. Apparatus according to Claim 1, **characterized in that** it has at least in sections a C-shaped configuration.

3. Apparatus according to Claim 2, **characterized in that** it has exclusively a C-shaped configuration.

4. Apparatus according to any of Claims 1 to 3, **characterized in that** the two curved, wire-shaped sections (24', 24", 26', 26") have at their ends a spherical element (27', 27").

5. Apparatus according to Claim 4, **characterized in that** the spherical elements (27', 27") are designed as metal spheres.

6. Apparatus according to any of Claims 1 to 5, **characterized in that** the housing (28) includes a unit for generating an electrical stimulus, and the two curved, wire-shaped sections (24', 24", 26', 26") each have in their end region an electrode (18, 19) for applying the stimulus to the skin surface.

7. Apparatus according to Claim 6, **characterized in that** the housing (28) and the two curved, wire-shaped sections (24', 24", 26', 26") are designed and disposable in such a way that the electrodes (18, 19) come to rest in the region of or near the tragus (4).

8. Apparatus according to Claim 7, **characterized in that** the two curved, wire-shaped sections (24', 24", 26', 26") are designed and disposable in such a way that they come to rest on the skin surface above the vagus nerve (nervus vagus).

9. Apparatus according to any of Claims 1 to 5, **characterized in that** the housing (28) comprises a unit for measuring a parameter, namely a physiological, a pathophysiological or a physical parameter, and the two curved, wire-shaped sections (24', 24", 26', 26") are designed as resilient holders.

10. Apparatus according to any of Claims 1 to 9, **characterized in that** the curved, wire-shaped sections (24', 24", 26', 26") consist of deformable material.

11. Apparatus according to any of Claims 1 to 10, **characterized in that** a rechargeable battery (22) is disposed in the apparatus.

## Revendications

1. Dispositif d'application transcutanée d'un stimulus ou de détection transcutanée d'un paramètre à travers la surface de la peau d'un animal vertébré, en particulier d'un être humain,
le dispositif (17) étant configuré de telle sorte qu'il peut être installé complètement dans le pinna (pavillon de l'oreille, 1) de l'animal vertébré,
**caractérisé en ce que**
le dispositif (17) présente un boîtier (28) qui contient l'électronique de commande et qui peut être placé entièrement dans le pavillon (1), deux tronçons incurvés, en forme de fil et à action élastique (24', 24", 26' , 26") étant disposés sur le boîtier (28) pour fixer le dispositif (17) par correspondance mécanique ou par frottement dans le pavillon (1).

2. Dispositif selon la revendication 1, **caractérisé en ce qu'**au moins certaines de ses parties ont la forme d'un C majuscule.

3. Dispositif selon la revendication 2, **caractérisé en ce qu'**il présente exclusivement la forme d'un C.

4. Dispositif selon l'une des revendications 1 à 3, **caractérisé en ce que** les deux tronçons incurvés en forme de fil (24', 24", 26', 26") présentent un élément sphérique (27', 27") à leurs extrémités.

5. Dispositif selon la revendication 4, **caractérisé en ce que** les éléments sphériques (27', 27") sont configurés comme billes métalliques.

6. Dispositif selon l'une des revendications 1 à 5, **caractérisé en ce que** le boîtier (28) contient un système de production d'un stimulus électrique et **en ce que** les deux tronçons incurvés en forme de fil (24', 24", 26', 26") présentent dans chacune de leur extrémité une électrode (18, 19) qui applique le stimulus sur la surface de la peau.

7. Dispositif selon la revendication 6, **caractérisé en ce que** le boîtier (28) et les deux tronçons incurvés en forme de fil (24', 24" , 26', 26") sont configurés et peuvent être disposés de telle sorte que les électrodes (18, 19) viennent se placer dans la zone du tragus (4) ou à proximité d'elle.

8. Dispositif selon la revendication 7, **caractérisé en ce que** les deux tronçons incurvés en forme de fil (24', 24", 26', 26") sont configurés et peuvent être disposés de telle sorte qu'ils viennent se placer sur la surface de la peau au-dessus du nerf vague (nervus vagus).

9. Dispositif selon l'une des revendications 1 à 5, **caractérisé en ce que** le boîtier (28) contient un système de détection d'un paramètre, à savoir un paramètre physiologique, un paramètre pathophysiologique ou un paramètre physique, et **en ce que** les deux tronçons incurvés en forme de fil (24', 24", 26', 26") sont configurés comme supports élastiques.

10. Dispositif selon l'une des revendications 1 à 9, **caractérisé en ce que** les tronçons incurvés en forme de fil (24', 24", 26', 26") sont constitués d'un matériau déformable.

11. Dispositif selon l'une des revendications 1 à 10, **caractérisé en ce qu'**une batterie rechargeable (22) est disposée dans le dispositif.
